# NEUE EUROPÄISCHE PATENTSCHRIFT

(11) **EP 1 140 235 B2**
(45) Veröffentlichungstag und Bekanntmachung des Hinweises auf die Entscheidung über den Einspruch: **19.12.2007**
(45) Hinweis auf die Patenterteilung: 28.08.2002
(21) Anmeldenummer: 99946186.6
(22) Anmeldetag: 17.09.1999
(51) Int. Cl.: A61L 24/10, A61L 15/32, A61L 15/42

(54) **FIBRINKLEBERGRANULAT UND VERFAHREN ZU DESSEN HERSTELLUNG**
FIBRIN-BASED GLUE GRANULATE AND CORRESPONDING PRODUCTION METHOD
GRANULES DE COLLE DE FIBRINE ET LEUR PROCEDE DE FABRICATION

(30) Priorität: 23.12.1998 DE 19859611; 21.06.1999 DE 19928372; 21.06.1999 DE 19928371
(43) Veröffentlichungstag der Anmeldung: 10.10.2001
(73) Patentinhaber: ZLB Behring GmbH, 35041 Marburg (DE)
(72) Erfinder: RAPP, Mirna, D-35037 Marburg (DE)
(86) Internationale Anmeldenummer: PCT/EP1999/006898
(87) Internationale Veröffentlichungsnummer: WO 2000/038752

(56) Entgegenhaltungen:
- WO-A-00/24436
- WO-A-97/28832
- WO-A-97/44015
- US-A- 4 427 651
- US-A- 4 453 939

## Beschreibung

Gegenstand der Erfindung ist ein rieselfähiges Fibrinklebergranulat, das alle zur Bildung eines stabilen Fibringels erforderlichen Substanzen enthält und direkt zur Wundverklebung eingesetzt werden kann. Es wird durch die Sprühtrocknung in der Wirbelschicht mittels eines Fluidisationsgases erzeugt.

Es ist bekannt, daß nach der Entstehung einer Wunde die Wundheilung durch eine Aktivierungskaskade mehrerer hintereinandergeschalteter Gerinnungsfaktoren eingeleitet wird. Daraus resultiert am Ende die Reaktion zwischen dem aktivierten Thrombin und Fibrinogen in Gegenwart von Kalziumionen zur Bildung einer Fibrinmatrix, die schließlich die Wunde abdeckt und somit eine Hämostase bewirkt. Diese Fibrinmatrix wird durch den aktivierten Faktor XIII (F XIIIa) über zusätzliche kovalente Bindungen weiter verfestigt, wodurch deren mechanische Stabilität und Resistenz gegen einen vorzeitigen proteolytischen Abbau erhöht wird.

In der modernen Chirurgie gewinnt die Hämostase durch Fibrinklebung zunehmend an Bedeutung, da es sich bei dem sogenannten Fibrinkleber um ein gut verträgliches und die Wundheilung förderndes Biomaterial handelt. Diese Methode eignet sich hervorragend zur Hämostase von stark blutenden Wunden bei Operationen an parenchymatösen, inneren Organen, bei Hauttransplantationen, in der Notfallchirurgie bei inneren und äußeren Verletzungen, vor allem aber auch zur unterstützenden Abdichtung von Nähten zur Vermeidung postoperativer Blutungen. In der HNO- und Gesichts-Chirurgie wird für die Heilung äußerer Wunden der Fibrinkleber dem Nahtverschluß aus kosmetischen Gründen vorgezogen. Auch wird der Fibrinkleber zunehmend in der endoskopischen Chirurgie z. B. zur Blutstillung von Magengeschwüren eingesetzt.

Die heute im Handel befindlichen Fibrinkleber wie Beriplast® enthalten neben anorganischen Salzen und Aminosäuren die aus dem Humanplasma gewonnenen Gerinnungsfaktoren Fibrinogen, Thrombin und Faktor XIII, zusätzlich aber auch Albumin und Fibronektin zur Förderung der Wundheilung. Obwohl das Präparat sehr gute biochemische und hämostatische Eigenschaften aufweist, bedarf es einer aufwendigen Vorbereitung vor seiner Anwendung. Die voneinander getrennten Fibrinogen- und Thrombinlyophilisate werden separat aufgelöst, in zwei voneinander getrennten Spritzen aufgezogen und in eine spezielle Halterung/Vorrichtung eingespannt. Dieses Verfahren erfordert neben viel zeit auch gut geschultes Personal. Eine Variante des Fibrinklebers wird als Tissucol® bereits in fertig gelöster Form in Spritzen in den Handel gebracht, ist jedoch nur bei tiefen Temperaturen von -20 °C lagerfähig und muß vor der Anwendung im Wasserbad aufgetaut werden. Somit finden beide Varianten des Fibrinklebers keine Anwendung in Situationen, wo ein gebrauchsfertiger und sofort ohne Vorbereitungen einsetzbarer Fibrinkleber von Nöten ist. Außerdem wäre ein gebrauchsfertiger und gut dosierbarer Fibrinkleber auch deshalb kostengünstiger, weil überschüssiges Material weder unnötig vorbereitet noch verworfen zu werden braucht.

Eine mögliche Verbesserung der Handhabung des Fibrinklebers könnte ein Einkomponentenkleber sein, der alle zur Bildung des Fibrins nötigen Komponenten in einem Kompartiment beinhaltet.

Die Entwicklung eines Einkomponentenklebers in einer wässrigen Lösung ist allerdings in der Praxis sehr schwer realisierbar. Eine Möglichkeit besteht allenfalls darin, die Komponenten des Fibrinklebers im getrockneten Zustand zu mischen, womit sich diese nach Applikation auf der Wunde in der Blutflüssigkeit oder dem Wundexsudat lösen und in situ eine Fibrinmatrix ausbilden, die eine Hämostase bewirkt. Dafür ist auch notwendig, das naturgemäß schwer lösliche Fibrinogen in eine derartige trockene Form zu bringen, aus der es rasch in Lösung geht und dabei sofort mit dem Thrombin reagiert.

Die Druckschriften WO 97/28832 und US-A- 4 453 939 offenbaren feine Pulver zur Stillung von Blutungen, welche Trombin, Faktor XIII, Fibrinogen und CaCl₂ enthalten.

Mischungen zur Hämostase auf Basis von Thrombin und Fibrinogen mit einer Partikelgröße von 0.1 bis 5 µm werden in US-A- 4 427 651 beschrieben.

Es hat auch schon Versuche gegeben, mit einem gezielten Lyophilisationsverfahren Partikel zu entwickeln, die Fibrinogen oder Thrombin enthalten und nach der Herstellung miteinander vermischt auf der Wunde aktiviert werden. So wird in der Internationalen Patentanmeldung WO 97/44015 die Herstellung sogenannter Mikropartikel auf Fibrinogen- oder Thrombinbasis beschrieben, die jeweils einzeln sprühgetrocknet werden. Diese Mikropartikel bestehen zu über 90 % aus Körnchen mit einer Größe bis zum 20 µm. Sie sollen gut löslich sein und können miteinander vermischt zur Wundheilung eingesetzt werden. Ein Nachteil dieser Mikropartikel ist allerdings, daß es sich dabei um ein sehr stark staubendes Pulver handelt, wodurch eine direkte Applikation wie das Aufstreuen auf eine Wunde nicht möglich ist. Ein derartiges Pulver benötigt daher ein spezielles Applikationssystem, was seine Handhabung und klinischen Indikationen drastisch einschränkt.

Es stellte sich deshalb die Aufgabe, ein Fibrinklebergranulat zu entwickeln, das gut löslich, rieselfähig und nicht staubend ist und damit direkt auf die Wunde aufgestreut werden kann z. B. nach dem Prinzip eines Salzstreuers.

Diese Aufgabe wird erfindungsgemäß durch ein rieselfähiges Fibrinklebergranulat gelöst, das Thrombin, Faktor XIII, Fibrinogen und ein Kalziumsalz in Granulatkörnchen mit einer Partikelgröße von 100 bis 200 µm enthält. Aufgrund dieser Partikelgröße ist das erfindungsgemäße Fibrinklebergranulat nicht staubend, gut löslich und rieselfähig und kann hervorragend auf eine Wundfläche oder ein feuchtes Gewebe aufgetragen werden, wo es sofort eine Fibrinmatrix ausbildet.

Einem derartigen Fibrinklebergranulat können auch noch Albumin, Fibronektin, Aminosäuren und physiologisch verträgliche anorganische Salze zugesetzt werden. Es kann außerdem als ein Freisetzungssystem für biologische, pflanzliche und/oder synthetische Faktoren verwendet werden. Diese Faktoren können die Wundheilung unterstützen oder als Antifibrinolytika, Antibiotika, Chemotherapeutika oder Immunmodulatoren wirken. Sie werden dem Fibrinklebergranulat während des Sprühtrocknungsprozesses zugesetzt.

Ein geeignetes Verfahrensprinzip für die Herstellung des erfindungsgemäßen Fibrinklebergranulats ist bereits aus der Internationalen Patentanmeldung WO 96/15849 bekannt. Dort ist ein Verfahren zur Trocknung von Blutplasma, Blutplasmafraktionen oder daraus gewonnen Blutplasmaprodukten beschrieben, bei dem das Behandlungsgut im flüssigen oder gelösten Zustand in einen evakuierbaren Behälter gesprüht wird, wodurch die Trocknung - bis zur Granulatform - mittels eines Fluidisatiionsgases in der Wirbelschicht durchgeführt wird. Auf Fibrinogen und Thrombin kann dieses Verfahren jedoch nicht ohne weiteres angewendet werden, da diese Substanzen bekannterweise nach Kontakt mit wässrigen Lösungen zu Fibrin reagieren. Deshalb kommt die Anwendung wässriger Lösungen für die Sprühtrocknung dieser Komponenten nicht in Frage. Um trotzdem beide Komponenten in einem Partikel zu erhalten, werden die Komponenten erfindungsgemäß in einem einzigen organischen Lösungsmittel zusammen suspendiert und daraus sprühgetrocknet. Fibrinogen, Thrombin und Faktor XIII lassen sich in organischen Lösungsmitteln wie den niederen Alkoholen, vorzugsweise Isopropanol oder Ethanol, Aceton, Nitrilen, flüssigen Karbonsäureestern, Ethern, Chloroform, Dimethylformamid und Dimethylsulfoxid auch in Gegenwart von CaCl₂ mehr oder weniger homogen suspendieren, ohne daß sie eine Reaktion zu Fibrin zeigen. Nach Entfernung des organischen Lösungsmittels sind sie in wässriger Phase wieder zur Fibrinbildung befähigt.

Erfindungsgemäß erfolgt die Sprühtrocknung dabei entweder durch das Top-Spray-Verfahren, bei dem die Flüssigkeit im Gegenstrom zum Fluidisationsgas geführt wird oder im Gleichstrom (Bottom-Spray-Verfahren). Durch das Einsprühen des flüssigen Behandlungsgutes in den evakuierbaren Behälter durch eine geeignete Düse wird eine feine Verteilung erreicht. Das Fluidisationsgas dient dabei sowohl zur Verwirbelung des zu behandelnden Gutes als auch zur Wärmeübertragung. Deshalb wird ein erwärmtes Gas als Fluidisationsgas eingesetzt. Durch die Messung der Produkttemperatur während des Wirbelschichtprozesses und einer darauf basierenden Prozeßsteuerung kann eine produktschonende Trocknung eingehalten werden. Als Fluidisationsgas kann entweder Luft oder ein Inertgas wie Stickstoff eingesetzt werden. Die Trocknung wird dabei so lange fortgeführt, bis das Behandlungsgut in fein verteilter Granulatform und einer Partikelgröße von 100 bis 200 µm vorliegt.

Das erfindungsgemäße Fibrinklebergranulat kann mit oder ohne ein in den evakuierbaren Behälter vorgelegtes Trägermaterial hergestellt werden. Als Trägermaterial kommen dabei vor allem Zucker und Zuckeralkohole wie Saccharose, Lactose oder Mannit in Betracht, die gut bioverträglich sind. Es können aber auch Proteine wie Serumalbumin als Trägermaterial eingesetzt werden. Besonders bevorzugt ist es, die Fibrinkleberkomponenten selbst, also Fibrinogen, Faktor XIII, Thrombin, CaCl₂ oder ihre Gemische in pulverförmigem Zustand als Trägermaterial einzusetzen, auf das die wässrige Lösung oder Suspension der Fibrinkleberkomponenten in organischen Lösungsmittel zwecks Bildung des Granulates aufgesprüht wird. Der Zusatz eines weiteren Trägermaterials wie einem Zucker, Mannit oder Albumin entfällt dann ganz.

Ein besonders bevorzugtes Verfahren besteht in einer zweistufigen Sprühtrocknung, bei der zuerst ein Fibrinogengranulat hergestellt wird. Dieses Granulat kann neben Fibrinogen auch andere Proteine, Kohlenhydrate, Aminosäuren und physiologisch verträgliche anorganische Salze, zusätzlich aber auch noch ein Kalziumsalz enthalten. Die Partikelgröße dieses Granulates beträgt 100 bis 200 µm. Auf dieses Fibrinogengranulat wird eine feine Thrombin-Suspension in einem organischen Lösungsmittel aufgesprüht, die gelöste Kalziumionen enthalten kann, wenn diese nicht schon dem Fibrinogengranulat zugesetzt waren. Die Konzentration der Kalziumionen beträgt 1 bis 100 mM, vorzugsweise 10 bis 50 mM. Man erhält auf diese Weise ein Fibrinklebergranulat mit einer Partikelgröße, die zwischen 100 und 200 µm liegt und eine körnige, sehr gut lösliche Struktur aufweist. Dabei entstehen keine kompakten Partikel wie kleine Kügelchen, sondern ein Granulat mit vielen feinen Kanälen. Dadurch erreicht man eine relativ große Partikelgröße, wodurch das Produkt gleichzeitig staubfrei und gut löslich wird, ähnlich wie der bekannte Instant-Kaffee. Dieses Granulat läßt sich hervorragend auf eine Wundfläche aufstreuen und bildet nach Kontakt mit einem wässrigen Medium sofort ein festes und elastisches Fibringel.

Das erfindungsgemäße Fibrinklebergranulat ist jedoch auch durch Sprühtrocknung von Fibrinogen-Konzentrat aus einer wässrigen Lösung auf eine Vorlage z. B. Mannit, erhältlich.

Dabei wird zunächst ein Fibrinogen/Mannit-Granulat erhalten, auf das dann anschließend Thrombin/Kalziumsalz, z. B. aus isopropanolischer Suspension, aufgesprüht wird. Das organische Lösungsmittel verhindert die Bildung von Fibrin nach Kontakt von Fibrinogen mit dem Thrombin.

Schließlich ist es aber auch möglich, separat Fibrinogen- und Thrombin-Granulate mit der vorstehend genannten Partikelgröße in getrennten Verfahren herzustellen, wobei beide Substanzen aus wässrigen Lösungen sprühgetrocknet werden können. Allerdings benötigt man dann bei der Herstellung des Thrombingranulates einen ausreichenden Anteil an Trägermaterial, da im Fibrinkleber die Menge an Thrombin gewichtsmäßig um den Faktor 10² bis 10³ kleiner ist als diejenige von Fibrinogen. Diese beiden Granulate werden miteinander vermischt und können dann entsprechend zur Hämostase und Wundheilung eingesetzt werden.

Die nach den vorstehend genannten Verfahren hergestellten Fibrinklebergranulate wurden anschließend auf ihre biomechanischen Eigenschaften untersucht und dabei die folgenden Ergebnisse erzielt:
Reisskraft nach in vitro Hautklebung (Klebefläche: 2,25 cm²)

Ergebnis einer Vergleichsstudie anhand einer Randomisierungsliste zu der Reisskraft des einheitlichen Granulates (Thrombin, Fibrinogen und Faktor XIII in einem Partikel), des Granulat-Gemisches (Fibrinogen-Granulat + Thrombin-Granulat) und des flüssigen Fibrinklebers (Beriplast®):

| Testsubstanz | Reisskraft |
|---|---|
| Einheitliches Granulat (Mischgranulat) | 3,3 N |
| Granulat-Gemisch | 1,8 N |
| Beriplast® | 1,5 N |

Die gemessenen Werte zeigen deutlich den Vorteil des einheitlichen Granulates (Mischgranulat) gegenüber dem Granulatgemisch bezüglich der biomechanischen Eigenschaften. Die Menge an aktiven Komponenten war in allen drei Testsubstanzen annähernd identisch.

Hinzu kommt, daß das erfindungsgemäße Fibrinklebergranulat sowohl bei Raumtemperatur als auch bei Temperaturen von 2 - 8°C mindestens 6 - 8 Monate lagerfähig ist, ohne daß die einzelnen Komponenten merklich an Aktivität verlieren.

Das erfindungsgemäße rieselfähige Fibrinklebergranulat unterscheidet sich durch eine einfachere Handhabung von den bisher bekannten Fibrinklebern, da keine Vorbereitungsmaßnahmen notwendig sind und es sich stets im gebrauchsfertigen Zustand befindet. Es ist deshalb ganz besonders für die Notfallchirurgie geeignet. Es hat außerdem den Vorteil der außerordentlich einfachen Anwendung durch Auftragen auf die Wundflächen nach der Art eines Salzstreuers. Es eignet sich hervorragend für chirurgische Anwendungen, bei denen eine rasche Hämostase durch Aufsaugen von Blut und gleichzeitige Fibrinklebung erreicht werden soll.

Obwohl mit dem vorstehend genannten Granulaten der Einsatz des Fibrinklebers erheblich erleichtert wird und aufwendige Operationsvorbereitungen unter Einsatz von speziellem Personal und entsprechenden Vorrichtungen verringert werden können, besteht weiterhin ein Bedarf an einfachen Fibrinkleberzubereitungen, die in jedem ärztlichen Notfallkoffer enthalten und an der Unfallstelle ohne längere Vorbereitungen sofort einsatzfähig sind.

Eine Lösung für dieses Problem konnte nun durch die Entwicklung eines Brausegranulats oder eines Brausepulvers gemäß den Anspruchen zur Erzeugung eines zur Blutstillung geeigneten Schaumes gefunden werden, das außer dem Granulatgemisch oder Mischgranulat, enthaltend Fibrinogen, Faktor XIII, Thrombin und ein lösliches Kalziumsalz, auch noch die zur CO₂-Bildung erforderlichen Substanzen enthält.

Das anspruchsgemäße Brausegranulat oder Brausepulver hat neben vielen anderen Vorteilen auch den Vorzug, daß durch das Aufschäumen die Granulatmasse aufgelockert und der Zutritt von Flüssigkeit in das Innere der Granulatkörnchen erleichtert wird. Es bildet sich dann sehr schnell ein stabiler Fibrinschaum aus, der die blutende Wunde abdeckt und die Blutung schnell zum Stillstand bringt. Dabei kann die Schaumbildung direkt auf der Wunde stattfinden, wobei die zur Schaumbildung erforderliche Feuchtigkeit durch das Wundsekret zur Verfügung gestellt wird. Die Schaumbildung kann aber auch zum Beispiel in einer Schale oder auf einem Teller durch Zugabe von Feuchtigkeit erfolgen und der fertig ausgebildete Schaum dann auf die blutende Wunde gelegt werden. Aufgrund seiner großen Flexibilität kann der so gebildete Schaum nicht nur zur äußerlichen Abdeckung von Wunden, sondern auch bei Blutungen von Operationswunden eingesetzt werden, wobei der Schaum in die blutende Operationswunde hineingestopft wird und sich dann auf das blutende Gewebe legt und damit eine schnelle Stillung der Blutung bewirkt.

Das anspruchsgemäße Brausegranulat oder Brausepulver kann in seinem therapeutischen Wert noch weiter verbessert werden, wenn ihm die Wundheilung fördernde biologische, pflanzliche oder synthetische Wirkstoffe wie Immunglobuline, Chemotherapeutika oder Antibiotika zugesetzt werden. Diese Substanzen werden bei der Herstellung des Granulats oder des Brausepulvers auf das rieselfähige, trockene Fibrinklebergranulat aufgesprüht oder mit ihm vermischt. Hieraus kann dann auch eine Brausetablette hergestellt werden, die in einer genau dosierten und leicht handhabbaren Form alle zur Herstellung eines zur Blutstillung geeigneten Schaumes enthält.

Im allgemeinen ist es ausreichend, das anspruchsgemäße Brausegranulat oder Brausepulver in einer Menge anzuwenden, die je nach der Größe der blutenden Wunde Fibrinogen in einer Menge von 0,1 bis 2,5 g und Thrombin in einer Menge von weniger als 10 I.E. enthält. Wird eine Brausetablette eingesetzt, können in diese auch Bruchrillen eingeprägt sein, die es ermöglichen, zur Blutstillung kleinerer Wunden einen Teil der Tablette abzubrechen, wenn die mit dem Tablettenteil erzeugte Schaummenge zur Stillung der Blutung bereits ausreicht.

Mit den anspruchsgemäße Granulatgemischen oder Mischgranulaten lassen sich darüber hinaus aber auch zur Blutstillung hervorragend einsetzbare galenische Zubereitungen herstellen, die in einfachster Weise anwendbar sind und auch bei Bedarf an der Unfallstelle sofort zur Verfügung stehen, ohne daß es langwieriger Vorbereitungen bedarf.

Dieses Ziel wird durch ein bioabbaubares Wundvlies erreicht, das auch bei großflächigeren Hautverletzungen Blutungen zum Stillstand bringt. Dabei wird ein Fibrinklebergranulat auf das aus einem bioabbaubaren Polymeren bestehende Trägermaterial entweder direkt oder zusammen mit einem biokompatiblen Hilfsstoff aufgetragen, in welchem der Fibrinkleber eingebettet ist. Als hierfür geeignetes Trägermaterial eignen sich vor allem natürliches oder chemisch modifiziertes Kollagen, Keratin, Gelatine, Kohlenhydrate oder Cellulosederivate. Das Trägermaterial kann auch aus einem synthetischen, bioabbaubaren Polymeren bestehen. Geeignet sind u.a. Polyhydroxycarbonsäuren, Polyester, Polycyanoacrylate, Polyaminosäuren, Polyalkohole sowie Silikone. Auf dieses Trägermaterial wird vorzugsweise eine Zubereitung aufgetragen, die das Fibrinogen in einer Menge von 0,05 bis 50 mg/cm², vorzugsweise 1 bis 20 mg/cm² sowie Thrombin in einer Menge von 1 µg bis 10 mg/cm², vorzugsweise 0,05 bis 2 mg/cm² enthält. Zur Verbesserung der Anhaftung können der Fibrinkleberzubereitung als Hilfsstoffe Polyethylenglykol (PEG) mit einer geeigneten Molekülgröße oder ein Gemisch mehrerer Polyethylenglykole unterschiedlicher Molekülgrößen zugesetzt werden.

Eine weitere Verbesserung bei der Blutstillung lässt sich dadurch erreichen, daß das beschriebene Wundvlies auf eine Bandage oder ein Pflaster aufgetragen wird. Dabei soll die Bandage an der zur Auflage auf die blutende Wunde vorgesehenen Stelle mit einem anspruchsgemäßen Wundvlies beschichtet sein. Zur Herstellung der anspruchsgemäßen Bandage werden Polyethylenglykol 4000 oder Polyethylenglykol 6000 oder deren Gemische bevorzugt eingesetzt. Dabei wird das Polyethylenglykol zur Herstellung der Beschichtungsmasse in einem organischen Lösungsmittel, bevorzugt Isopropanol, gelöst, das in einer Konzentration von 0,5 bis 70%, bevorzugt in einer Konzentration von 5 bis 30% (w/v) eingesetzt wird. Das Fibrinklebergranulat wird auf der Bandage ausgebreitet und dann mit der Isopropanol-Polyethylenglykol 6000-Lösung benetzt. Nach dem Abdampfen des organischen Lösungsmittels erhält man ein bioabbaubares Wundvlies, welches eine gut haftende Fibrinkleberbeschichtung besetzt. Das organische Lösungsmittel eignet sich zur Beschichtung deshalb hervorragend, weil es leicht abdampfbar ist, die Reaktion zu Fibrin unterbindet und die Erhaltung der Aktivität der einzelnen Komponenten gewährleistet. Außerdem bleibt die Granulatform nach Behandlung im organischen Lösungsmittel, bevorzugt Isopropanol, erhalten.

Im allgemeinen wird das anspruchsgemäße Wundvlies nur einseitig mit der beschriebenen blutungsstillenden, salbenoder gelartigen Zubereitung versehen. Es gibt allerdings auch Anwendungsfälle, in denen eine zweiseitige Beschichtung des Wundvlieses vorzuziehen ist. Wird die Wunde mit einer derartigen Bandage umwickelt, dann entfaltet sich die blutstillende Wirkung des Fibrinklebers unmittelbar auf der Wunde, sobald unter der Einwirkung des Wundsekrets aus den in der Bandagenauflage enthaltenen Bestandteilen das Fibrin gebildet wird. Die Anwendung kann in vielen Fällen noch dadurch erleichtert werden, daß das anspruchsgemäße Wundvlies auf ein zur Pflasterfertigung geeignetes, wasserdichtes oder wasserdurchlässiges Flächenmaterial aufgetragen wird, wobei an den Rändern des Pflasters Klebeflächen freibleiben, die mit einem physiologisch gut verträglichen Klebstoff beschichtet sind. Mit einem derartigen Pflaster lässt sich die blutende Wunde schnell, einfach und dauerhaft bedecken und bewirkt eine alsbaldige Blutstillung.

Eine Blutstillung lässt sich in einfachster Weise auch durch eine salben- oder gelartige Zubereitung erreichen, die aus einer hydrophilen, wasserfreien Salbengrundlage besteht, in die Partikel eines Fibrinklebers eingebettet sind. Als hydrophile wasserfreie Salbengrundlage eignen sich ganz besonders Polyole, zum Beispiel Polyethylenglykole, Polypropylenglykole oder Ethylen-Propylencopolymere, in denen die Partikel des Fibrinklebers gleichmäßig verteilt sind, und die die im Wundsekret enthaltene Feuchtigkeit aufnehmen. Aus den Bestandteilen des Fibrinklebers bildet sich dann bei Feuchtigkeitszutritt sofort ein Fibringeflecht, das die Wunde schnell und wirksam abdeckt und die Blutung zum Stillstand bringt. Es liegt auf der Hand, daß für diese Einsatzweck fetthaltige und wasserabstoßende Salbengrundlagen nicht geeignet sind.

Der in den anspruchsgemäßen Zubereitungen enthaltene Fibrinkleber enthält eine trockene Mischung von Fibrinogen, Faktor XIII, Thrombin und einem löslichen Kalziumsalz. Die Zubereitung kann zweckmäßigerweise auch in eine Salbentube abgefüllt werden und ist dann über längere Zeit haltbar und kann in dieser Form unmittelbar eingesetzt werden.

Die Wirksamkeit der vorstehend beschriebenen, zur Blutstillung geeigneten Zubereitungen ist natürlich nur dann gewährleistet, wenn vor ihrer Anwendung der Zutritt von wässrigen Flüssigkeiten und damit eine vorzeitige Fibrinbildung vermieden wird. Hierauf ist auch schon bei der Herstellung der Zubereitungen Rücksicht zu nehmen. Dabei werden die anspruchsgemäßen Granulatgemische oder Mischgranulate mit der hydrophilen, aber wasserfreien Salbengrundlage in an sich bekannter Weise angeteigt. Die hierbei entstehende salben- oder gelartige Zubereitung kann dann auf das bioabbaubare Trägermaterial zur Herstellung eines Wundvlieses aufgestrichen oder direkt verwendet werden.

Eine weitere Verbesserung der anspruchsgemäßen Zubereitungen lässt sich erreichen, wenn außer dem Fibrinkleber weitere, die Wundheilung fördernde biologische, pflanzliche oder synthetische Wirkstoffe wie Immunglobuline, Chemotherapeutika oder Antibiotika zugesetzt werden.

Das anspruchsgemäße Wundvlies, die Bandage oder das Pflaster oder die salben- opder gelartige Zubereitung können zur Hämostase innerer oder äußerer Wunden in einfachster Weise wirkungsvoll eingesetzt werden.

Die Erfindung wird durch die nachfolgenden Beispiele erläutert.

### Beispiel 1

### Herstellung von Fibrinogen-Granulat ohne vorgelegtes Trägermaterial

Eine 10 %ige Proteinlösung von Beriplast®-Fibrinogenkonzentrat (enthält auch F XIII) wurde nach dem Top-Spray-Verfahren in Wirbelschicht sprühgetrocknet. Dieses Verfahren wurde in einer GPCG 1-Anlage der Firma Glatt GmbH durchgeführt und ist in der Internationalen Patentanmeldung WO 96/15849 beansprucht und detailliert beschrieben. Die Bedingungen sind:

| | |
|---|---|
| Eingangstemperatur | 37 °C |
| Ausgangstemperatur | 30 °C |
| Sprühdruck | 3,0 bar |
| Sprührate | 3,2 g/min |

Das derart hergestellte Fibrinogen-Granulat hatte eine mittlere Partikelgröße von 100 µm und war sehr gut löslich. Analytische Aktivitätsmessungen haben gezeigt, daß die Aktivität von Fibrinogen und F XIII durch den Sprühtrocknungsprozeß bei diesen Bedingungen nicht beeinträchtigt wird.

### Beispiel 2

### Herstellung von Fibrinogen-Granulaten mit vorgelegtem Trägermaterial

200 g Mannit oder Albumin wurden in die Sprühtrocknungskammer vorgelegt. Auf die Vorlage wurde 100 g Fibrinogenkonzentrat in Wirbelschicht unter folgenden Bedingungen aufgesprüht:

| | |
|---|---|
| Eingangstemperatur | 30 °C |
| Ausgangstemperatur | 24 °C |
| Sprühdruck | 2,5 bar |
| Sprührate | 3,0 - 8,0 g/min |

Es resultierte ein rieselfähiges und gut lösliches Granulat mit einer mittleren Partikelgröße von 100 µm, bei dem die Fibrinogen- und F XIII-Aktivität voll wiederfindbar ist.

### Beispiel 3

### Herstellung von Fibrinkleber-Granulat

Auf das in Beispiel 1 oder 2 hergestellte Fibrinogen-Granulat wurde eine isopropanolische Thrombin/CaCl₂-Suspension aufgesprüht. Der Prozeß lief unter folgenden Bedingungen ab:

| | |
|---|---|
| Eingangstemperatur | 30 °C |
| Ausgangstemperatur | 25 °C |
| Sprühdruck | 2,5 bar |
| Sprührate | 3,0 - 8,0 g/min |

Das auf diese Weise hergestellte Fibrinkleber-Granulat mit einer mittleren Partikelgröße von 100 µm war rieselfähig, staubte nicht und bildete sofort nach Kontakt mit einer wässrigen Lösung ein stabiles, d.h. durch F XIII kovalent vernetztes Fibringerinnsel.

### Beispiel 4

### Herstellung von Thrombin-Granulat

Auf eine Vorlage von Mannit oder Humanserumalbumin wurde eine wässrige 0,3 %ige Thrombin-Lösung aufgesprüht. Die Bedingungen waren wie folgt:

| | |
|---|---|
| Eingangstemperatur | 30 °C |
| Ausgangstemperatur | 23 °C |
| Sprühdruck | 2,5 bar |
| Sprührate | 4,2 g/min |

Die mittlere Partikelgröße des gebildeten Granulates betrug ca. 65 µm. Es war rieselfähig und nicht staubend. Es ließ sich gut mit dem Fibrinogen-Granulat mischen und war auch als Fibrinkleber einsetzbar.

### Beispiel 5

### Herstellung eines Fibrinkleber-Granulats aus einer alle Fibrinkleberkomponenten enthaltenden isopropanolischen Suspension

Entsprechend den Beispielen 1 und 2 wurde in eine Sprühtrockungskammer, in der entweder überhaupt kein Trägermaterial vorgelegt war oder in der sich ein Trägermaterial wie Mannit, Albumin oder eine oder mehrere pulverförmige Fibrinkleberkomponenten befand, eine isopropanolische Suspension gesprüht, die alle Fibrinkleberkomponenten, also Fibrinogen, Faktor XIII, Thrombin, CaCl₂ oder ihrer Gemische enthielt, und anschließend in der Wirbelschicht sprühgetrocknet. Der Prozess lief unter folgenden Bedingungen:

| | |
|---|---|
| Eingangstemperatur | 30° C |
| Ausgangstemperatur | 25° C |
| Sprühdruck | 2,5 bar |
| Sprührate | 3,0 bis 8,0 g/min |

Das auf diese weise hergestellte Fibrinklebergranulat mit einer mittleren Partikelgröße von etwa 100 µm war rieselfähig und staubte nicht und bildete sofort nach Kontakt mit einer wässrigen Lösung ein stabiles, vernetztes Fibringerinnsel.

### Beispiel 6

### Herstellung einer mit Fibrinkleber beschichteten bioabbaubaren Bandage

Auf ein 50 x 50 mm² Ethisorb® Patch Typ 6 (Ethicon GmbH) werden 250 mg Fibrinkleberpulver bzw. -granulat aufgetragen und gleichmäßig verteilt (=10 mg Fibrinkleberpulver bzw. - granulat pro cm²) verteilt. Dann werden auf die Beschichtung insgesamt 2,5 ml einer Lösung von Isopropanol/20% PEG 6000 gleichmäßig aufgesprüht. Nach Abdampfen des Isopropanols erhält man eine bioabbaubare Bandage, bestehend aus einem Trägermaterial und einer gut haftenden Fibrinkleberbeschichtung, die beim Biegen der Bandage nicht abbröckelt.

### Beispiel 7

### Herstellung einer mit Fibrinkleber beschichteten bioabbaureen Bandage

Auf ein 20 x 30 mm² großes Vicryl-Vlies (Ethicon GmbH) werden 60 mg Fibrinkleberpulver bzw. -granulat aufgetragen und gleichmäßig verteilt. (= 10 mg Pulver/cm²). Dann werden auf die Beschichtung insgesamt 0,6 ml einer Lösung von Isopropanol/20% PEG 6000 gleichmäßig aufgesprüht. Nach dem Abdampfen des Isopropanols wird eine flexible, bioabbaubare Bandage mit einer gut haftenden Fibrinkleberbeschichtung erhalten.

### Beispiel 8

### Mit Fibrinkleber beschichtetes Kollagenvlies

Das Kollagenvlies Interceed (Johnson & Johnson), Größe 50 x 50 mm², wird mit 250 mg Fibrinkleberpulver bzw. -granulat gleichmäßig versetzt. Dann werden auf die Beschichtung insgesamt 0,6 ml einer Lösung von Isopropanol/10% PEG 6000 gleichmäßig aufgesprüht. Nach Abdampfen des Isopropanols erhält man ein Fibrinkleber-kombiniertes Kollagenvlies.

## Patentansprüche

1. Rieselfähiges Fibrinkleber-Granulat, **dadurch gekennzeichnet, daß** es Granulatkörnchen mit einer Partikelgröße von 100 bis 200 µm aufweist, die Thrombin, Faktor XIII, Fibrinogen und ein Kalziumsalz enthalten.

2. Fibrinkleber-Granulat nach Anspruch 1, **dadurch gekennzeichnet, daß** es zusätzlich Albumin, Fibronektin und/oder weitere, die Wundheilung fördernde Substanzen enthält.

3. Brausegranulat oder Brausepulver zur Erzeugung eines zur Blutstillung geeigneten Schaumes, **dadurch gekennzeichnet, daß** es rieselfähiges Fibrinkleber-Granulat, das Granulatkörnchen mit einer Partikelgröße von über 50 bis etwa 1000 µm aufweist, die Thrombin, Faktor XIII, Fibrinogen und ein Kalziumsalz enthalten, und zusätzlich zur CO₂-Bildung erforderliche Substanzen enthält.

4. Brausegranulat oder Brausepulver nach Anspruch 3, **dadurch gekennzeichnet, daß** es zur CO₂-Bildung eine Mischung aus einem Carbonat und einer physiologisch verträglichen organischen Säure enthält.

5. Zubereitung zur Stillung von Blutungen, **dadurch gekennzeichnet, daß** es ein aus einem bioabbaubaren Trägermaterial bestehendes Wundvlies enthält, welches mit einem rieselfähigen Fibrinkleber-Granulat, das Granulatkörnchen mit einer Partikelgröße von über 50 bis etwa 1000 µm aufweist, die Thrombin, Faktor XIII, Fibrinogen und ein Kalziumsalz enthalten, beschichtet ist.

6. Zubereitung nach Anspruch 5, **dadurch gekennzeichnet, daß** das Wundvlies mit einer hydrophilen, wasserfreien Salbengrundlage beschichtet ist, in die das Fibrinkleber-Granulat eingebettet ist.

7. Wundvlies nach den Ansprüchen 5 und 6, **dadurch gekennzeichnet, daß** das bioabbaubare Trägermaterial aus natürlichem oder chemisch modifiziertem Collagen, Keratin, Gelatine, Kohlenhydraten oder Cellulosederivaten besteht.

8. Wundvlies nach den Ansprüchen 5 und 6, **dadurch gekennzeichnet, daß** das bioabbaubare Trägermaterial aus einem Polymeren aus der Gruppe der Polyhydroxycarbonsäuren, der Polyester, der Polycyanoacrylate, der Polyaminosäuren, der Polyalkohole oder der Silikone besteht.

9. Wundvlies nach den Ansprüchen 5 bis 8, **dadurch gekennzeichnet, daß** es Fibrinogen in einer Menge von 0,05 bis 50 mg/cm² und Thrombin in einer Menge von 1 µg bis 20 mg/cm² enthält.

10. Wundvlies nach den Ansprüchen 5 bis 9, **dadurch gekennzeichnet, daß** die den Fibrinkleber enthaltende Zubereitung einseitig oder beidseitig auf das Trägermaterial aufgetragen ist.

11. Bandage, **dadurch gekennzeichnet, daß** sie an der zur Auflage auf die blutende Wunde vorgesehenen Stelle mit einem Wundvlies der Ansprüche 5 bis 10 beschichtet ist.

12. Pflaster, **dadurch gekennzeichnet, daß** es aus einem wasserdichten oder wasserdurchlässigen Flächenmaterial besteht, das an der zur Auflage auf die blutende Wunde vorgesehenen Stelle mit einem Wundvlies der Ansprüche 5 bis 10 beschichtet ist und an den Rändern Klebeflächen aufweist.

13. Zubereitung zur Stillung von Blutungen, **dadurch gekennzeichnet, daß** sie aus einer hydrophilen, wasserfreien Salbengrundlage besteht, in die Partikel eines Fibrinklebers, die eine Partikelgröße von über 50 bis etwa 1000 µm aufweisen, und die Thrombin, Faktor XIII, Fibrinogen und ein Kalziumsalz enthalten, eingebettet sind.

14. Verfahren zur Herstellung eines rieselfähigen Fibrinkleber-Granulates mit einer Partikelgröße von über 50 bis etwa 1000 µm, das Thrombin, Faktor XIII, Fibrinogen und ein Kalziumsalz enthält, **dadurch gekennzeichnet, daß** alle Bestandteile des Fibrinklebers in einem organischen Lösungsmittel suspendiert und in einem evakuierbaren Behälter mittels eines Fluidisationsgases in der Wirbelschicht bis zu einer Partikelgröße von über 50 bis 1000 µm, vorzugsweise 100 bis 200 µm, sprühgetrocknet werden.

15. Verfahren nach Anspruch 14, **dadurch gekennzeichnet, daß** es mit oder ohne ein in den Behälter vorgelegtes Trägermaterial hergestellt wird.

16. Verfahren zur Herstellung eines Fibrinkleber-Granulates mit einer Partikelgröße von 100 bis 200 µm, das Thrombin, Faktor XIII, Fibrinogen und ein Kalziumsalz enthält, **dadurch gekennzeichnet, daß** zunächst ein Fibrinogen-Granulat hergestellt wird, auf welches eine Suspension eines organischen Lösungsmittels enthaltend Thrombin aufgesprüht wird, wobei entweder dem Fibrinogen-Granulat oder der Thrombin-Lösung ein Kalziumsalz zugesetzt wird.

17. Verfahren zur Herstellung eines Fibrinkleber-Granulates mit einer Partikelgröße von 100 bis 200 µm, das Thrombin, Faktor XIII, Fibrinogen und ein Kalziumsalz enthält, **dadurch gekennzeichnet, daß** die separat hergestellten Fibrinogen- und Thrombin-Granulatkörnchen, die jeweils eine Partikelgröße von 100 bis 200 µm aufweisen, miteinander vermischt werden.

18. Verfahren zur Herstellung einer Zubereitung nach den Ansprüchen 5 bis 13, **dadurch gekennzeichnet, daß** der als Granulatgemisch oder als Mischgranulat vorliegende Fibrinkleber auf ein bioabbaubares Trägermaterial aufgeschichtet wird.

19. Verfahren zur Herstellung der Zubereitung von Anspruch 13, **dadurch gekennzeichnet, daß** ein als Granulatgemisch oder als Mischgranulat vorliegender Fibrinkleber mit der hydrophilen, wasserfreien Salbengrundlage angeteigt wird.

20. Verfahren zur Herstellung einer Zubereitung nach den Ansprüchen 5 bis 13, **dadurch gekennzeichnet, daß** dem Fibrinklebergranulat weitere die Wundheilung fördernde, biologische, pflanzliche oder synthetische Wirkstoffe wie Immungloboline, Chemotherapeutika oder Antibiotika zugesetzt werden.

21. Fibrinkleber-Granulat nach den Ansprüchen 1 bis 4 oder einer Zubereitung nach den Ansprüchen 5 bis 13, **dadurch gekennzeichnet, daß** es zur Wundheilung in der Chirurgie, der Gewebstherapie und/oder als Trägermaterial für biologische Faktoren eingesetzt wird.

22. Wundvlies, Bandage, Pflaster oder Salbe oder gelartige Zubereitung der Ansprüche 5 bis 13 zur Hemostase innerer oder äußerer Wunden.

23. Verwendung eines Brausegranulats oder eines Brausepulvers der Ansprüche 3 und 4 zur Herstellung einer Brausetablette durch Verpressen.

## Claims

1. A pourable granular fibrin adhesive material, **characterized in that** it contains grains of granular material having a particle size of over 50 to approximately 1000 µm, which contain thrombin, factor XIII, fibrinogen and a calcium salt as a unitary granular material.

2. The granular fibrin adhesive material according to claim 1, **characterized in that** the grains of granular material have a particle size of 100 to 200 µm.

3. The granular fibrin adhesive material according to claims 1 and 2, **characterized in that** it additionally contains albumin, fibronectin and/or further substances promoting wound healing.

4. An effervescent granular material or effervescent powder for the production of a foam which is suitable for haemostasis, **characterized in that**, in addition to the pourable granular fibre adhesive material of claims 1 to 3, it also contains substances necessary for CO₂ formation.

5. The effervescent granular material or effervescent powder, according to claim 4, **characterized in that** it contains a mixture of a carbonate and a physiologically tolerable organic acid for CO₂ formation.

6. A preparation for staunching haemorrhages, **characterized in that** it contains a nonwoven wound material consisting of a biodegradable carrier material, which is coated with a pourable granular fibrin adhesive material of claims 1 to 3.

7. The preparation according to claim 6, **characterized in that** the nonwoven wound material is coated with a hydrophilic, anhydrous ointment base in which the fibrin adhesive of claims 1 to 3 is embedded.

8. The nonwoven wound material according to claims 6 and 7, **characterized in that** the biodegradable carrier material consists of natural or chemically modified collagen, keratin, gelatine, carbohydrates or cellulose derivatives.

9. The nonwoven wound material according to claims 6 and 7, **characterized in that** the biodegradable carrier material consists of a polymer from the group consisting of the polyhydroxycarboxylic acids, the polyesters, the polycyanoacrylates, the polyamino acids, the polyalcohols and the silicones.

10. The nonwoven wound material according to claims 6 to 9, **characterized in that** it contains fibrinogen in an amount from 0.05 to 50 mg/cm² and thrombin in an amount from 1 µg to 20 mg/cm².

11. The nonwoven wound material according to claims 6 to 10, **characterized in that** the preparation comprising the fibrin adhesive is applied to the carrier material on one side or both sides.

12. A bandage, **characterized in that** it is coated, on the site intended for laying on the haemorrhaging wound, with a nonwoven wound material of claims 6 to 11.

13. A plaster, **characterized in that** it consists of a waterproof or water-permeable surface material which is coated, on the site intended for laying on the haemorrhaging wound, with a nonwoven wound material of claims 6 to 11 and has adhesive areas on the edges.

14. A preparation for staunching haemorrhages, **characterized in that** it consists of a hydrophilic, anhydrous ointment base in which the particles of a fibrin adhesive of claims 1 to 3 are embedded.

15. A process for the production of the granular fibrin adhesive material of claims 1 to 3, **characterized in that** all constituents of the fibrin adhesive are suspended in an organic solvent and spray dried in an evacuable container by means of a fluidization gas in the fluidized bed up to a particle size of over 50 to 1000 µm, preferably 100 to 200 µm.

16. The process according to claim 15, **characterized in that** the granular fibrin adhesive material is prepared with or without a carrier material introduced into the container.

17. A process for the production of a fibrin adhesive according to claims 1 to 3, **characterized in that** a granular fibrinogen material is initially prepared, onto which is sprayed a suspension of an organic solvent comprising thrombin, a calcium salt being added either to the granular fibrinogen material or the thrombin solution.

18. A process for the production of a preparation according to claims 6 to 14, **characterized in that** the fibrin adhesive, which is present as blend granules, is coated onto biodegradable carrier material.

19. A process for the production of the preparation of claim 14, **characterized in that** it comprises forming a fibrin adhesive which is present as blend granules into a paste with the hydrophilic, anhydrous ointment base.

20. A process for the production of a preparation according to claims 6 to 14, **characterized in that** further biological, plant or synthetic active compounds promoting wound healing, such as immunoglobulins, chemotherapeutics or antibiotics, are added to the granular fibrin adhesive material.

21. The granular fibrin adhesive material according to claims 1 to 5 or a preparation according to claims 6 to 14, for wound healing in surgery, tissue therapy and/or as a carrier material for biological factors.

22. The nonwoven wound material, bandage, plaster or ointment or gelatinous preparation of claims 6 to 14 for the haemostasis of internal or external wounds.

23. The use of effervescent granules or an effervescent powder of claims 4 and 5 for the production of an effervescent tablet by compression.

## Revendications

1. Produit granulé pour colle de fibrine, pouvant s'écouler, **caractérisé en ce qu'**il comporte des granules ayant une taille de particule de plus de 50 à environ 1 000 µm, qui contiennent sous forme de produit granulé homogène de la thrombine, du facteur XIII, du fibrinogène et un sel de calcium.

2. Produit granulé pour colle de fibrine selon la revendication 1, **caractérisé en ce que** les granules ont une taille de particule de 100 à 200 µm.

3. Produit granulé pour colle de fibrine selon les revendications 1 et 2, **caractérisé en ce qu'**il contient en outre de l'albumine, de la fibronectine et/ou d'autres substances facilitant la cicatrisation.

4. Produit granulé effervescent ou poudre effervescente pour la production d'une mousse appropriée à l'arrêt du saignement, **caractérisé**(e) en ce qu'en plus du produit granulé pour colle de fibrine, pouvant s'écouler, des revendications 1 à 3, il/elle contient également encore des substances requises pour la formation de CO₂.

5. Produit granulé effervescent ou poudre effervescente selon la revendication 4, **caractérisé**(e) en ce qu'il/elle contient pour la formation de CO₂ un mélange d'un carbonate et d'un acide organique physiologiquement acceptable.

6. Préparation pour l'arrêt de saignements, **caractérisée en ce qu'**elle contient un non-tissé chirurgical constitué d'un matériau de support biodégradable, qui est revêtu d'un produit granulé pour colle de fibrine, pouvant s'écouler, des revendications 1 à 3.

7. Préparation selon la revendication 6, **caractérisée en ce que** le non-tissé chirurgical est revêtu d'une base de pommade anhydre hydrophile, dans laquelle est incluse la colle de fibrine des revendications 1 à 3.

8. Non-tissé chirurgical selon les revendications 6 et 7, **caractérisé en ce que** le matériau de support biodégradable est constitué de collagène naturel ou modifié chimiquement, kératine, gélatine, glucides ou dérivés de cellulose.

9. Non-tissé chirurgical selon les revendications 6 et 7, **caractérisé en ce que** le matériau de support biodégradable est constitué d'un polymère choisi dans le groupe des poly(acide hydroxycarboxylique)s, des polyesters, des polycyanoacrylates, des poly(aminoacide)s, des polyalcools ou des silicones.

10. Non-tissé chirurgical selon les revendications 6 à 9, **caractérisé en ce qu'**il contient du fibrinogène en une quantité de 0,05 à 50 mg/cm² et de la thrombine en une quantité de 1 µg à 20 mg/cm².

11. Non-tissé chirurgical selon les revendications 6 à 10, **caractérisé en ce que** la préparation contenant la colle de fibrine est appliquée d'un côté ou des deux sur le matériau de support.

12. Pansement **caractérisé en ce que**, sur la partie prévue pour l'application sur la plaie saignante, il est revêtu d'un non-tissé chirurgical des revendications 6 à 11.

13. Emplâtre **caractérisé en ce qu'**il consiste en un matériau plan perméable ou imperméable à l'eau qui, sur la partie prévue pour l'application sur la plaie saignante, est revêtu d'un non-tissé chirurgical des revendications 6 à 11 et présente sur les bords des surfaces adhésives.

14. Préparation destinée à l'arrêt de saignements, **caractérisée en ce qu'**elle est constituée d'une base de pommade anhydre, hydrophile, dans laquelle sont incluses des particules d'une colle de fibrine des revendications 1 à 3.

15. Procédé pour la préparation du produit granulé pour colle de fibrine des revendications 1 à 3, **caractérisé en ce que** tous les composants de la colle de fibrine sont mis en suspension dans un solvant organique et séchés par atomisation dans un récipient pouvant être mis sous vide, au moyen d'un gaz de fluidisation, dans le lit fluidisé, jusqu'à une taille de particule de plus de 50 à 1 000 µm, de préférence, de 100 à 200 µm.

16. Procédé selon la revendication 15, **caractérisé en ce que** le produit granulé est préparé avec ou sans un matériau de support disposé au préalable dans le récipient.

17. Procédé pour la préparation d'une colle de fibrine selon les revendications 1 à 3, **caractérisé en ce qu'**on prépare d'abord un produit granulé contenant du fibrinogène, sur lequel on pulvérise une suspension d'un solvant organique contenant de la thrombine, un sel de calcium étant ajouté soit au produit granulé contenant du fibrinogène, soit à la solution de thrombine.

18. Procédé pour la production d'une préparation selon les revendications 6 à 14, **caractérisé en ce que** la colle de fibrine, se trouvant sous forme de produit granulé mélangé, est appliquée sur un matériau de support biodégradable.

19. Procédé pour la production de la préparation selon la revendication 14, **caractérisé en ce qu'**une colle de fibrine, se trouvant sous forme de produit granulé mélangé, est transformée en pâte avec la base de pommade anhydre, hydrophile.

20. Procédé pour la production d'une préparation selon les revendications 6 à 14, **caractérisé en ce qu'**on ajoute au produit granulé pour colle de fibrine d'autres substances actives biologiques, végétales ou synthétiques, facilitant la cicatrisation, telles que des immunoglobulines, des agents chimiothérapeutiques ou des antibiotiques.

21. Produit granulé pour colle de fibrine selon les revendications 1 à 5, ou d'une préparation selon les revendications 6 à 14, **caractérisé en ce qu'**il est utilisé pour la cicatrisation en chirurgie, l'histothérapie et/ou en tant que matériau de support pour des facteurs biologiques.

22. Non-tissé chirurgical, pansement, emplâtre ou pommade ou préparation de type gel des revendications 6 à 14, pour l'hémostase de plaies internes ou externes.

23. Utilisation d'un produit granulé effervescent ou d'une poudre effervescente des revendications 4 et 5, pour la fabrication par compression d'un comprimé effervescent.
